# EUROPEAN PATENT SPECIFICATION

(11) **EP 1 658 504 B1**
(45) Date of publication and mention of the grant of the patent: **05.08.2009**
(21) Application number: 04764350.7
(22) Date of filing: 20.08.2004
(51) Int. Cl.: G01N 33/68, C12Q 1/48

(54) **INHIBITION OF S6 KINASE ACTIVITY FOR THE TREATMENT OF INSULIN RESISTANCE**
HEMMUNG DER S6-KINASE-AKTIVITÄT ZUR BEHANDLUNG VON INSULINRESISTENZ
INHIBITION DE L'ACTIVITE DE LA KINASE S6 POUR TRAITER LA RESISTANCE A L'INSULINE

(30) Priority: 22.08.2003 US 497226 P
(43) Date of publication of application: 24.05.2006
(73) Proprietor: Novartis Forschungsstiftung, Zweigniederlassung, 4058 Basel (CH)
(72) Inventor: AUWERX, Johan, F-67150 Hindisheim (FR); FRIGERIO, Francesca, CH-4058 Basel (CH); FUMAGALLI, Stefano, CH-4058 Basel (CH); KOZMA, Sara, Wyoming, OH 45215 (US); PICARD, Frederic, Levis, QC, G6V 4A8 (CA); STICKER-JANTSCHEFF, Melanie, 79211 Denzlingen (DE); THOMAS, George, F-68220 Hésingue (FR); UM, Sung, Hee, CH-4058 Basel (CH); WATANABE, Mitsuhiro, F-67400 Illkirch-Graffenstaden (FR)
(74) Representative: Roth, Peter Richard
(86) International application number: PCT/EP2004/009368
(87) International publication number: WO 2005/019829

(56) References cited:
- WO-A-03/012032
- US-A1- 2003 143 656
- EL-HASCHIMI KARIM ET AL: "Insulin resistance and lipodystrophy in mice lacking ribosomal S6 kinase 2." DIABETES, vol. 52, no. 6, June 2003 (2003-06), pages 1340-1346, XP002301362 ISSN: 0012-1797

## Description

The current invention relates to insulin resistance and diabetes, in particular to the treatment of insulin resistance or diabetes with modulators of S6 kinase (S6K) activity.

### BACKGROUND OF THE INVENTION

Type II diabetes is the most common form of diabetes in the Western world and is strongly linked to obesity - over 80% of sufferers are obese. In patients with Type II diabetes, insulin is less able to promote the uptake of glucose into muscle and fat, a state termed insulin resistance. The molecular basis by which obesity leads to impaired insulin action are not well understood.

Insulin normally acts to maintain glucose homeostasis by regulating its own production and secretion by pancreatic beta cells, and by controlling glucose utilization in peripheral tissues. Recent studies have implicated the mTOR / S6 kinase signal transduction pathway in this process. S6 kinase is a kinase that phosphorylates the ribosomal protein, S6. In particular, S6K1 (also known as p70/p85 S6 kinase) deficient mice are mildly glucose intolerant (hyperglycaemic) and hypoinsulinemic, not due to a lesion in glucose sensing or insulin production, but to a reduction in pancreatic endocrine mass, which is accounted by a selective decrease in beta-cell. S6K1 deficient mice maintain normal fasting glucose levels, suggesting they may be hypersensitive to insulin in their peripheral tissues (Pende et al., 2000, Nature, 408, 994-997).

This phenotype is reminiscent of a form of preclinical type 2 diabetes mellitus, where protein malnutrition-induced hypoinsulinaemia predisposes individuals to glucose intolerance. A limited period of protein malnutrition in rats also leads to mild glucose intolerance arising from a persistent decrease in β cell size and insulin secretion, an effect partially attenuated by mild insulin hypersensitivity in peripheral tissues (Swenne et al., 1992, Diabetologia 35, 939-945; Swenne et al., J. Endocrinol. 118, 295-302; Grace et al., 1990, Diabetes Metab. 16, 484-491 (1990).

S6K1-deficient mice are viable and fertile but exhibit a conspicuous reduction in body size during embryogenesis, an effect mostly overcome by adulthood. A comparison of homozygous mutant mice at 3.5 weeks of age demonstrated that the weights of all organs were proportional to the reduction in body weight. The small size of the homozygous mutant mice is consistent with a defect in translational capacity (Shima et al., 1998, EMBO J., 17, 6649-6659). As S6K1 deficient mice reach maturity, the difference in weight that they display at birth, as compared to wild type mice, diminishes from 20% to 15%. Such mice might accumulate fat and become insulin resistant as a function of age and an increase in dietary fat, as would wild type mice.

The role of S6 kinase activity in insulin resistance has been addressed in human studies. Insulin-sensitive and insulin-resistant, non-diabetic Pima Indians were treated with insulin over 2 hours. Although basal levels of S6 kinase activity were similar for both groups, S6 kinase was activated only three-fold in insulin resistant individuals compared to five fold in insulin-sensitive individuals, suggesting an impaired S6 kinase activity in insulin resistant individuals.

S6K activity has previously been implicated in cancer and angiogenesis. WO93/19752 describes the use of rapamycin and its derivatives as inhibitors of p70 S6 kinase and their use to inhibit proliferation or the immune response of a cell. US 2003/0083284 describes antisense compounds for the inhibition of expression of p70 S6 kinase (referring to both the 70kDa and nuclear, 85kDa isoforms). The antisense nucleic acids are proposed to be potentially useful in treating infections, inflammation and tumor formation, as well as metabolic disorders. US2003/0143656 proposes that compounds capable of increasing the activity of p70 S6K may be useful in treating diabetes or obesity, or may be useful in inhibiting apoptosis.

Attoub et al. (2001, Faseb J., 14, 2329-2338) show that rapamycin blocks leptin function, in particular leptin-induced invasion of cells into collagen gels (as a model of carcinogenesis). Leptin therapy has been used to promote weight loss while preserving lean mass in obese patients with congenital leptin deficiency, suggesting leptin in the treatment of obesity or diabetes.

There remains a need to provide new targets and to develop new medicaments for the treatment of insulin resistance, in particular Type II diabetes (also referred to as non-insulin dependent diabetes mellitus, NIDDM) and this invention meets that need.

### SUMMARY OF THE INVENTION

In accordance with a first aspect of the invention, a method of identifying an agent effective in treating insulin resistance is provided, the method comprising the steps of: i) incubating S6 kinase with a compound; ii) detecting S6 kinase activity; and iii) determining a compound-induced inhibition in the S6 kinase activity relative to when the compound is absent, wherein an inhibition of the S6 kinase activity in the presence of the compound is indicative of an agent effective in treating insulin resistance. The compound -induced inhibition is preferably independent of an effect of mammalian Target of Rapamycin (mTOR) activity. In one embodiment, the modulation is inhibition of S6 kinase 1 activity. S6 kinase activity can be conveniently assayed using S6 as a substrate and is easily amenable to high throughput assays.

Also provided by the invention are methods of screening for an agent effective in treating insulin resistance, comprising contacting transcriptionally active cellular components with a nucleic acid encoding an S6K gene operably linked to a promoter sequence or an S6K promoter sequence operably linked to a reporter gene in the presence of at least one compound; and detecting an effect of the compound on S6 kinase expression or S6 kinase promoter activity, wherein detection of a decrease in S6 kinase expression or promoter activity is indicative of an agent effective in treating insulin resistance. Such assays can be cell-based assays, where the transcriptionally active cellular components and nucleic acid is present in a cell. In preferred embodiments, the S6 kinase is S6 kinase 1.

Thus, also provided are specific inhibitors of S6 kinase for the manufacture of a medicament for the treatment or prophylactic treatment of insulin resistance according to claim 8.

In a further aspect of the invention, a method of diagnosing a predisposition to insulin resistance or diabetes is provided, comprising: in a sample from an mammal, detecting the level of S6 kinase activity, and correlating a change in S6 kinase activity in the sample when compared to a normal control value or range of values with a predisposition to insulin resistance or diabetes, wherein an increase in S6 kinase 1 activity when compared to a normal control value or range of values is indicative of a predisposition to insulin resistance or diabetes.

### DETAILED DESCRIPTION OF THE INVENTION

There remains a need for more effective therapies to treat insulin resistance and Type II diabetes in individuals, particularly in obese individuals. The present inventors have discovered that in contrast to previously published studies, S6K1 activation results in insulin resistance thereby providing S6K1 as a pharmaceutical target for treating diabetes and related maladies through inhibition of S6K1 activity. Although mTOR (mammalian target of rapamycin, which phosphorylates and activates S6 kinase) can be targeted to modulate S6K1 activity, direct targeting of S6K1 avoids more general side-effects of inhibiting mTOR activity and provides more specificity for the treatment of patients with insulin resistance. In particular, mTOR is known to activate both S6K1 and S6K2, whereas the present inventors have found that the selective inhibition of S6K1 is desirable.

Accordingly, the present invention provides a method of identifying an agent effective in treating insulin resistance or diabetes (in particular high fat diet or obesity induced conditions), based on the modulation of S6 kinase activity, in particular S6 kinase 1 activity. Typically such a method will comprise the steps of incubating S6 kinase with a compound; detecting S6 kinase activity; and determining the compound-induced modulation in the S6 kinase activity relative to when the compound is absent. An inhibition of the S6 kinase activity in the presence of the compound is indicative of an agent effective in treating insulin resistance or diabetes. A control assay in the absence of the compound can be run in parallel.

Unless otherwise clear from the context, "S6K" or "S6 kinase" is used herein to encompass both S6K1 and S6K2 (see, for example, Genebank Accession No. M57428, AJ007938, AB019245, NM003952 and related sequences), although S6K1 is preferred. Exemplary functional equivalents (variants) or derivatives of S6K include molecules where S6K is covalently modified by substitution, chemical, enzymatic, or other appropriate means with a moiety other than a naturally occurring amino acid.

Generally speaking such variants will be substantially homologous to the 'wild type' or other sequence specified herein i.e. will share sequence similarity or identity therewith. Similarity or identity may be at the nucleotide sequence and/or encoded amino acid sequence level, and will preferably, be at least about 50%, 60%, or 70%, or 80%, most preferably at least about 90%, 95%, 96%, 97%, 98% or 99%. Sequence comparisons may be made using FASTA and FASTP (see Pearson & Lipman, 1988. Methods in Enzymology 183: 63-98). Parameters are preferably set, using the default matrix, as follows: Gapopen (penalty for the first residue in a gap): -12 for proteins / -16 for DNA; Gapext (penalty for additional residues in a gap): -2 for proteins / -4 for DNA; KTUP word length: 2 for proteins / 6 for DNA. Analysis for similarity can also be carried out using hybridisation. One common formula for calculating the stringency conditions required to achieve hybridization between nucleic acid molecules of a specified sequence homology is: Tm = 81.5oC + 16.6Log [Na+] + 0.41 (% G+C) - 0.63 (% formamide) - 600/#bp in duplex (Molecular Cloning: a Laboratory Manual: 2nd edition, Sambrook et al., 1989, Cold Spring Harbor Laboratory Press).

Variants that retain common structural features can be fragments of S6K, in particular fragments maintaining catalytic activity or isoform specific characteristics. For example, the carboxy- terminal sequences of S6K1 and S6K2 exhibit only about 20% identity and it therefore may be useful to include such isoform-specific features in fragments when isoform-specific assays are desired. Similarly, S6K1 can be phosphorylated at T447, which is absent in S6K2 providing an alternative or additional isoform-specific feature. Preferably, fragments will be between 50 and 350 amino acids in length.

Variants of S6K also comprise mutants thereof, which may contain amino acid deletions, additions or substitutions, subject to the requirement to maintain at least one feature characteristic of S6K, preferably catalytic activity and/or isoform-specific features as described above. Thus, conservative amino acid substitutions may be made substantially without altering the nature of S6K, as may truncations. Additions and substitutions may moreover be made to the fragments of S6K used in the screening methods of the invention, in particular those enhancing S6K catalytic activity or providing some other desirable property. For example, T389, T229 and S371 in mouse S6K1 (also known as p70S6K) are homologous to T389, T238 and S380 in Drosophila p70S6K. T389 is particularly indicated for mutation to an acidic amino acid residue in order to produce a constitutively active kinase. Fusion proteins with the S6K or S6K fragments referred to above may also be desirable.

The screening assays of the invention are not limited to any particular method of determining S6 kinase activity. S6 kinase assays are well known in the art (see for example USPN 6,372,467, which is herein incorporated by reference in its entirety). Briefly, S6 kinase will be incubated with a suitable substrate, such as S6, in a buffer allowing phosphorylation of S6. Phosphorylation of the substrate can be detected using a labelled phosphate group, such as the use of the radioactive label ³²P present as the ATP source in the buffer. Alternatively, antibodies specific for the phosphorylated products of S6K catalytic activity can be used to detect activity. As will be apparent to those of ordinary skill in the art, the assays are easily amenable to high through-put technologies using robotics and automated processes.

Alternatively, the S6 kinase activity can be assayed using a synthetic substrate, such as those comprising Arg- (Arg)-Arg-X-X-Ser-X (e.g., KRRRLASLAA or KRRRLSSLRASTSKSESSQK) (Flowtow and Thomas (1992) J. Biol. Chem. 267: 3074-3078.

S6K activity can also be assayed by detecting downstream targets of the kinase. For example, S6K is known to affect transcription and translation of specific targets, such as genes with polypyrimidine tracts (5'TOPs) and ribosomal genes. (Fumagalli S, Thomas G. (1999) Ribosmal Protein S6 Phosphorylation and Signal Transduction. In: Translational Control. Eds. Hershey, J, Mathews, M, Sonenberg, N. Cold Spring Harbor Press. pp 695-717).

Thus, in accordance with a further aspect of the invention, a method is provided for screening an agent effective in treating insulin resistance, by identifying compounds that decrease expression of an S6K gene or a gene expressed under the control of S6K regulatory sequences.

Such methods comprise contacting transcriptionally active cellular components, preferably in a cell, with a nucleic acid encoding an S6K gene operably linked to a promoter sequence or an S6K promoter sequence (or other S6K regulatory regions allowing expression of the reporter gene) operably linked to a reporter gene in the presence of at least one compound; and detecting an effect of the compound on expression of the coding region, be it S6 kinase expression or reporter gene expression. Expression of S6 kinase can be detected at the transcript level (for example, by hybridization using specific probes or PCR) or at the protein level (for example, using an antibody). A decrease in S6 kinase expression or promoter activity is indicative of an agent effective in treating insulin resistance. Such assays can be cell-based assays, where the transcriptionally active cellular components and nucleic acid is present in a cell, although in vitro transcription assays are also well known in the art. In preferred embodiments, the S6 kinase is S6 kinase 1.

The reporter gene encodes any molecule capable of providing a detectable change. Such reporter molecules include fluorescent moieties (e.g., fluorescent proteins, such as, cyan fluorescent protein, CFP; yellow fluorescent protein, YFP; blue fluorescent protein, BFP; or green fluorescent protein, GFP; all available commercially, Clontech Living Colors User Manual, antigens, reporter enzymes and the like. Reporter enzymes include, but are not limited to, the following: beta-galactosidase, glucosidases, chloramphenicol acetyltransferase (CAT), glucoronidases, luciferase, peroxidases, phosphatases, oxidoreductases, dehydrogenases, transferases, isomerases, kinases, reductases, deaminases, catalases and urease. In selecting a reporter molecule to be used in the presently claimed method, the reporter molecule itself should not be inactivated by any putative agent or other component present in the screening assay, including inactivation by any protease activity present in the assay mixture. The selection of an appropriate reporter molecule will be readily apparent to those skilled in the art.

The nucleic acid will typically be provided in a vector allowing replication in one or more selected host cells, as is well known for a variety of bacteria, yeast, and mammalian cells. For example, various viral origins (SV40, polyoma, adenovirus, VSV or BPV) are useful for cloning vectors in mammalian cells. The vector may, for example, be in the form of a plasmid, cosmid, viral particle, phage, or any other suitable vector or construct which can be taken up by a cell and used to express the sequence of interest or reporter gene.

Expression vectors usually contain a promoter operably linked to the protein-encoding nucleic acid sequence of interest, so as to direct mRNA synthesis. Promoters recognized by a variety of potential host cells are well known, as are the S6K1 and S6K2 promoters (upstream regulatory sequences). "Operably linked" means joined as part of the same nucleic acid molecule, suitably positioned and oriented for transcription to be initiated from the promoter. DNA operably linked to a promoter is "under transcriptional control" of the promoter. Transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g. the actin promoter or an immunoglobulin promoter, and from heat-shock promoters, provided such promoters are compatible with the host cell systems. Expression vectors of the invention may also contain one or more selection genes, such as genes conferring resistance to antibiotics or other toxins.

The methods of the invention may therefore further include introducing the nucleic acid into a host cell. The introduction, which may (particularly for in vitro introduction) be generally referred to without limitation as "transformation", may employ any available technique. For eukaryotic cells, suitable techniques may include calcium phosphate transfection, DEAE-Dextran, electroporation, liposome-mediated transfection and transduction using retrovirus or other virus, e.g. vaccinia, as is well known in the art. See, for example, Keown et al., Methods in Enzymology, 185:527 537 (1990) and Mansour et al., Nature 336:348-352 (1988).

Host cells transfected or transformed with expression or cloning vectors described herein may be cultured in conventional nutrient media. The culture conditions, such as media, temperature, pH and the like, can be selected by the skilled artisan without undue experimentation. In general, principles, protocols, and practical techniques for maximizing the productivity of cell cultures can be found in "Mammalian Cell Biotechnology: a Practical Approach", M. Butler, ed. JRL Press, (1991) and Sambrook et al, supra.

Assays specific for S6K1 can also be designed by detecting binding of specific proteins, such as nerabin to the C-terminal domain of S6K1, as is well known in the art (Burnett PE, Blackshaw S, Lai MM, Qureshi IA, Burnett AF, Sabatini DM, Snyder SH. Neurabin is a synaptic protein linking p70 S6 kinase and the neuronal cytoskeleton. Proc Natl Acad Sci U S A. 1998 Jul 7;95(14):8351-6).

Thus, in another embodiment of the invention the inhibition of the interaction of S6K1 with a binding partner is assessed. This may comprise (i) contacting S6K1 with a binding partner thereof in the presence and absence of a test substance; and (ii) determining whether the presence of a test substance inhibits the interaction between S6K1 and its binding partner.

Methods for assessing the interaction between a polypeptide and a binding partner may be any of the methods known to those skilled in the art and are disclosed here. Any of these methods can be used to assess whether a test substance inhibits the interaction between a polypeptide (in this case S6K1) and a binding partner.

In one embodiment, assays are those based upon S6K1 and its interaction with neurabin and comprise the step of determining whether the test substance inhibits the interaction between S6K1 and neurabin. This may be achieved by detecting the physical association between S6K1 and its binding partner through labelling one with a detectable label and bringing it into contact with the other which has been immobilised on a solid support. Suitable detectable labels include ³⁵S-methionine which may be incorporated into recombinantly produced S6K1 and/or the binding partner thereof. The recombinantly produced S6K1 and/or binding partner may also be expressed as a fusion protein containing an epitope which can be labelled with an antibody. Alternatively, double-labelling may be used as is well known in the art, for example, using a radioactive label and a scintillant.

Generally, a protein which is immobilized on a solid support may be immobilized using an antibody against that protein bound to a solid support or via other technologies which are known per se. A preferred in vitro interaction may utilise a fusion protein including a tag, such as glutathione-S-transferase (GST) or His6. The tag may be immobilized by affinity interaction, for example on glutathione agarose beads or Ni-matrices, respectively.

In an in vitro assay format of the type described above the putative inhibitor compound can be assayed by determining its ability to modulate the amount of labelled S6K1 or binding partner which binds to the immobilized binding partner, e.g., GST-binding partner or GST-S6K1 as the case may be. This may be determined by fractionating the glutathione-agarose beads by SDS-polyacrylamide gel electrophoresis. Alternatively, the beads may be rinsed to remove unbound protein and the amount of protein which has bound can be determined by counting the amount of label present in, for example, a suitable scintillation counter.

Alternatively an antibody attached to a solid support and directed against one of S6K1 or the binding partner may be used in place of GST to attach the molecule to the solid support. Antibodies against S6K1 and its binding partners may be obtained in a variety of ways known as such in the art. In an alternative mode, one of S6K1 and its binding partner may be labelled with a fluorescent donor moiety and the other labelled with an acceptor, which is capable of reducing the emission from the donor. This allows an assay according to the invention to be conducted by fluorescence resonance energy transfer (FRET). In this mode, the fluorescence signal of the donor will be altered when S6K1 and its binding partner interact. The presence to a candidate inhibitor that modulates the interaction will increase the amount of fluorescence signal of the donor.

FRET is a technique known per se in the art and thus the precise donor and acceptor molecules and the means by which they are linked to S6K1 and its binding partner may be accomplished by reference to the literature.

Suitable fluorescent donor moieties are those capable of transferring fluorogenic energy to another fluorogenic molecule or part of a compound and include, but are not limited to, coumarins and related dyes such as fluoresceins, rhodols and rhodamines, resorufins, cyanine dyes, bimanes, acridines, isoindoles, dansyl dyes, aminophthalic hydrazines such as luminol and isoluminol derivatives, aminophthalimides, aminonaphthalimides, aminobenzofurans, aminoquinolines, dicyanohydroquinones, and europium and terbium complexes and related compounds.

Suitable acceptors include, but are not limited to, coumarins and related fluorophores, xanthenes such as fluoresceins, rhodols and rhodamines, resorufins, cyanines, difluoroboradiazaindacenes, and phthalocyanines.

A preferred donor is fluorescein and preferred acceptors include rhodamine and carbocyanine. The isothiocyanate derivatives of these fluorescein and rhodamine, available from Aldrich Chemical Company Ltd, Gillingham, Dorset, UK, may be used to label S6K1 and its binding partner. For attachment of carbocyanine, see for example Guo et al, J. Biol. Chem., 270; 27562-8, 1995.

Assays of the invention may also be performed in vivo. Such an assay may be performed in any suitable host cell, e.g. a bacterial, yeast, insect or mammalian host cell. Yeast and mammalian host cells are particularly suitable. To perform such an assay in vivo, constructs capable of expressing S6K1 and its binding partner and a reporter gene construct may be introduced into the cells. This may be accomplished by any suitable technique, for example calcium phosphate precipitation or electroporation. The constructs may be expressed transiently or as stable episomes, or integrated into the genome of the host cell.

In vivo assays may also take the form of two-hybrid assays. Two-hybrid assays may be in accordance with those disclosed by Fields and Song, 1989, Nature 340; 245-246. In such an assay the DNA binding domain (DBD) and the transcriptional activation domain (TAD) of the yeast GAL4 transcription factor are fused to the first and second molecules respectively whose interaction is to be investigated. A functional GAL4 transcription factor is restored only when two molecules of interest interact. Thus, interaction of the molecules may be measured by the use of a reporter gene operably linked to a GAL4 DNA binding site, which is capable of activating transcription of said reporter gene. Other transcriptional activator domains may be used in place of the GAL4 TAD, for example the viral VP16 activation domain.

Irrespective of the form of assay used, they will typically be run with suitable controls routine to those of skill in the art and is preferably used to screen compounds that may be present in small molecule libraries, peptide libraries, phage display libraries or natural product libraries. Putative or actual inhibitors or other modulators may be provided from any source which it is desired to screen, and may or may not be naturally occurring or synthetic, and may or may not be peptides or polypeptides (e.g. antibodies) or nucleic acids (e.g. siRNA). Preferred inhibitors most suited for therapeutic applications will be small molecules e.g. from a combinatorial library such as are now well known in the art (see e.g. Newton (1997) Expert Opinion Therapeutic Patents, 7(10): 1183-1194). Preferred candidate substances may include small molecules such as PKC inhibitors.

A compound-induced modulation of S6K activity means that there is a change in S6K activity (enzymatic activity, signalling activity to downstream targets, promoter activity or expression) in the presence of the compound relative to when the compound is absent. In particular a compound induced inhibition of S6K activity is reflected by a decrease in S6K activity relative to when the compound is absent. Conversely, a compound induced activation of S6 activity is reflected by an increase in S6K activity.

Activators and inhibitors are referred to collectively herein as modulators and preferably influence the kinase activity of S6K directly. Assays carried out using reconstituted components can be easily designed to achieve direct S6K inhibition (i.e., specific inhibition of S6K catalytic activity and not inhibition of the formation of active kinase, for example, through the action of mTOR). Typically, S6 kinase 1 activity will be selectively inhibited (i.e., preferentially over S6 kinase 2 activity and other kinases and enzymes.), in particular when inhibition of S6 kinase 1 signaling and treatment of insulin resistance or diabetes is desired. Alternatively, S6 kinase 2 activity will be specifically activated for the same purpose.

S6K inhibitors are compounds that reduce S6K activity, e.g., S6K1 or S6K2 activity. For example, compounds that inhibit S6K enzymatic activity typically bind to an ATP binding site in S6K or bind to a catalytic domain of S6K. The compound preferentially inhibits S6K1 compared to S6K2 or other S6K isoforms, given the difference in phenotypes observed between S6K1 and S6K2 knock out mice. Thus, although compounds that inhibit S6K2 or both S6K1 and S6K2 (such as rapamycin, its derivatives or other mTOR inhibitors) may be useful, the selective inhibition of S6K1 is desirable for the treatment of insulin resistance or diabetes. Therefore, S6K1 inhibitors will typically reduce S6K1 activity by at least 10%, more preferably 20%, 50%, 100% and 200% compared to the level of reduction of S6K2 activity. Control assays may therefore be carried out, for example with immunoprecipitated S6K2 and compared to immunoprecipitated S6K1 to establish the selectivity of a modulator.

The screening methods of the invention may optionally further comprise a functional assay, comprising detecting an effect on insulin resistance. Suitable methods are set out in Examples below.

The screening methods may optionally include the step of administering a potential modulator to a non-human animal having an S6 kinase gene and determining whether insulin resistance is affected relative to when the compound is absent, for example an effect on insulin sensitivity upon high fat feeding of the animal. The non-human animals will typically be laboratory mammals such as mice or rats and various doses can be administered orally mixed with feed or by any other appropriate means, which may be chosen dependent on the properties of the compound, such as stability and targeted delivery. The S6 kinase gene may be from a different species as the laboratory mammal, for example, the use of a mouse comprising a human S6K gene, which replaces the mouse S6K gene, will be particularly useful to determine the effects of agents on human S6K without using human subjects.

The potency and efficacy of compounds for inhibition of S6K1 can also be assessed using an animal model for insulin resistance and compared with S6K1 knock out animals.

Kits useful for screening such compounds may also be prepared in accordance with the invention, and will comprise essentially S6K or a fragment thereof useful for screening, and instructions. Typically the S6K polypeptide will be provided together with means for detecting S6K activity and at least one compound (putative agent) or other substance described herein useful for carrying out the screening methods.

S6K for use in kits according to the invention may be provided in the form of a protein, for example in solution, suspension or lyophilised, or in the form of a nucleic acid sequence permitting the production of S6K or a fragment thereof in an expression system, optionally in situ.

Compounds (e.g., putative agents) may be inorganic or organic, for example, an antibiotic, antibody, polypeptide or peptide, and are typically isolated or purified. An "isolated" or "purified" composition is substantially free of cellular material or other contaminating proteins from the cell or tissue source from which it is derived, or substantially free from chemical precursors or other chemicals when chemically synthesized. A polypeptide that is substantially free of cellular material includes preparations of the polypeptide in which the polypeptide is separated from cellular components of the cells from which it is isolated, e.g., the polypeptide is recombinantly produced. Preferably, a preparation of a therapeutic compound, e. g., an S6K inhibitor, is at least 75%, more preferably 80%, more preferably 85%, more preferably 90%, more preferably 95%, more preferably 98%, and most preferably 99 or 100% of the dry weight of the preparation. Mixtures of compounds may also be tested during initial stages of screening.

Compounds may therefore include antibodies, preferably monoclonal antibodies, that are specific for S6K, in particular S6K1, in the sense of being able to distinguish between the polypeptide it is able to bind and other polypeptides of the same species for which it has no or substantially no binding affinity (e.g. a binding affinity of at least about 1000x worse). Specific antibodies bind an epitope on the molecule that is either not present or is not accessible on other molecules. For example, for isoform-specific inhibition (selective inhibition of S6K1 activity over S6K2 activity), the antibody may interfere with the C-terminal domain of S6K, which is not highly conserved between S6K1 and S6K2. Antibodies may be obtained using techniques which are standard in the art. For example, antibodies may be obtained from immunised animals using any of a variety of techniques known in the art, and screened, preferably using binding of antibody to antigen of interest. For instance, Western blotting techniques or immunoprecipitation may be used (Armitage et al, Nature, 357:80-82, 1992).

As an alternative or supplement to immunising a mammal with a peptide, an antibody specific for a protein may be obtained from a recombinantly produced library of expressed immunoglobulin variable domains, e.g. using lambda bacteriophage or filamentous bacteriophage which display functional immunoglobulin binding domains on their surfaces; for instance see WO92/01047.

Antibodies may be modified in a number of ways and include antibody fragments, derivatives, functional equivalents and homologues of antibodies, including synthetic molecules and molecules whose shape mimics that of an antibody enabling it to bind an antigen or epitope. Example antibody fragments, capable of binding an antigen or other binding partner are the Fab fragment consisting of the VL, VH, CI and CH1 domains; the Fd fragment consisting of the VH and CH1 domains; the Fv fragment consisting of the VL and VH domains of a single arm of an antibody; the dAb fragment which consists of a VH domain; isolated CDR regions and F(ab')2 fragments, a bivalent fragment including two Fab fragments linked by a disulphide bridge at the hinge region. Single chain Fv fragments are also included.

Humanized antibodies in which CDRs from a non-human source are grafted onto human framework regions, typically with the alteration of some of the framework amino acid residues, to provide antibodies which are less immunogenic than the parent non-human antibodies, are also included within the present invention.

As is apparent to one of skill in the art, a monoclonal antibody may be subjected to the techniques of recombinant DNA technology to produce other antibodies or chimeric molecules that retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP-A-184187, GB-A-2188638 or EP-A-0239400. Cloning and expression of chimeric antibodies are described in EP-A-0120694 and EP-A-0125023.

Peptides can also be used as inhibitors of S6K activity, such as a synthetic peptide containing a putative autoinhibitory domain (S6 kinase 1 residues 400-432; Flowtow and Thomas, 1992) or indeed the synthetic substrates referred to above (e.g., S6 230-249, Ala 235), which can further be used to model new inhibitors.

Alternatively, S6K activity in a cell can be reduced using nucleic acids, e.g. by pre- or post-transcriptional silencing. Thus, S6K sequences (in particular sequences specific to S6K1) may be inserted into the vectors as described above in an antisense orientation in order to provide for the production of antisense RNA or ribozymes.

Nucleic acid sequences selective for S6K1 over S6K2 include those encoding amino acids 33 -77 and amino acids 454-525 (numbering refers to that of SEQ ID NO:3 in US2003/0083284, which is hereby incorporated by reference) or portions thereof, which are typically at least 15, 18 or more nucleotides in length. Sequence comparisons may be made essentially as described above using FASTA and FASTP (see Pearson & Lipman, 1988. Methods in Enzymology 183: 63-98) to establish specificity of the sequences.

An alternative to anti-sense is to use double stranded RNA (dsRNA), which has been found to be even more effective in gene silencing than antisense alone (Fire A. et al Nature, Vol 391, (1998)). dsRNA mediated silencing is gene specific and is often termed RNA interference (RNAi) (See also Fire (1999) Trends Genet. 15: 358-363, Sharp (2001) Genes Dev. 15: 485-490, Hammond et al. (2001) Nature Rev. Genes 2: 1110-1119 and Tuschl (2001) Chem. Biochem. 2: 239-245).

RNA interference is a two-step process. First, dsRNA is cleaved within the cell to yield short interfering RNAs (siRNAs) of about 21-23nt length with 5' terminal phosphate and 3' short overhangs (-2nt). The siRNAs target the corresponding mRNA sequence specifically for destruction (Zamore P.D. Nature Structural Biology, 8, 9, 746-750, (2001). Thus in one embodiment, the inhibition is achieved using double stranded RNA comprising an S6K-encoding sequence, in particular a sequence selective for S6K1, which may for example be a double stranded RNA (which will be processed to siRNA, e.g., as described above). Cellular defense mechanisms, such as the PKR pathway will typically need to be circumvented, for example using siRNA directed against the individual components. These RNA products may be synthesised in vitro, e.g., by conventional chemical synthesis methods.

However, to avoid the PKR pathway, chemically synthesized siRNA duplexes of about 21-23 nucleotides in length with 3'-overhang ends are preferably used (Zamore PD et al Cell, 101, 25-33, (2000)). Synthetic siRNA duplexes have been shown to specifically suppress expression of endogenous and heterologeous genes in a wide range of mammalian cell lines (Elbashir SM. et al. Nature, 411, 494-498, (2001)).

Thus, siRNA duplexes containing between 20 and 25 bps, more preferably between 21 and 23 bps, of the S6K sequence, in particular sequences selective for S6K1 over S6K2, include form one aspect of the invention e.g. as produced synthetically, optionally in protected form to prevent degradation.

Alternatively siRNA may be produced from a vector, in vitro (for recovery and use) or in vivo. Accordingly, the vector may comprise a nucleic acid sequence encoding S6K (including a nucleic acid sequence encoding a variant or fragment thereof), suitable for introducing an siRNA into the cell in any of the ways known in the art, for example, as described in any of references cited herein, which references are specifically incorporated herein by reference.

In one embodiment, the vector may comprise a nucleic acid sequence according to the invention in both the sense and antisense orientation, such that when expressed as RNA the sense and antisense sections will associate to form a double stranded RNA. This may for example be a long double stranded RNA (e.g., more than 23nts), which may be processed in vitro with Dicer to produce siRNAs (see for example Myers (2003) Nature Biotechnology 21:324-328) or siRNA, hairpin structures. Alternatively, the sense and antisense sequences are provided on different vectors. These vectors and RNA products are useful, for example, to inhibit de novo production of the S6K polypeptide in a cell. Such nucleic acids and vectors can be introduced into a host cell or administered to a mammal in a suitable form.

Thus, nucleic acids, such as siRNA can be administered to inhibit S6K1 activity. SiRNA technology can be routinely applied based on sequences specific for S6K1, such as AGTGTTTGACATAGACCTG or preferably AAGGGGGCTATGGAAAGGTTT. Targeted expression of siRNAs can be achieved using tissue-specific promoters, such as promoters specific for adipose tissue, muscle, liver or other tissues mediating insulin resistance and glucose homeostasis.

For ease of administration, however, the compound is preferably a small molecule, which might bind to the catalytic site or ATP binding site. For isoform-specific inhibition, the compound may interfere with the C-terminal domain of S6K, which is not highly conserved between S6K1 and S6K2.

In order to potentially improve S6K modulators, isolated S6K can be used to establish secondary and tertiary structure of the whole protein or at least of the areas responsible for the enzymatic activity. Conventional methods for the identification of the 3-dimensional structure are, for example, X-ray studies or NMR studies. The data obtained with these or comparable methods may be used directly or indirectly for the identification or improvement of modulators of S6K, such as to provide selectivity between S6K1 and S6K2. A commonly used method in this respect is, for example, computer aided drug design or molecular modelling.

Compounds according to the invention may be identified by screening using the techniques described hereinbefore, and prepared by extraction from natural or genetically modified sources according to established procedures, or by synthesis, especially in the case of low molecular weight chemical compounds. Proteinaceous compounds may be prepared by expression in recombinant expression systems, for example a baculovirus system, or in a bacterial system. Proteinaceous compounds are mainly useful for research into the function of signalling pathways, although they may have a therapeutic application, such as humanized inhibitory antibodies directed against S6 kinase 1.

Low molecular weight compounds, on the other hand, are preferably produced by chemical synthesis according to established procedures. They are primarily indicated as therapeutic agents. PKC inhibitors, or derivatives or modifications thereof, may be used as potential agents effective in selectively inhibiting S6K1 and in treating insulin resistance or diabetes. Low molecular weight compounds and organic compounds in general may be useful as agents for use in the treatment of insulin resistance or diabetes.

The present invention also provides a method for reducing insulin resistance comprising contacting a cell, in vitro in particular myocytes, adipocytes and/or hepatocytes with an effective amount of an S6 kinase 1 inhibitor according to claim 7. Thus, also provided by the invention are compounds that directly modulate S6 kinase activity for use in treating insulin resistance and diabetes. In particular, compounds that selectively inhibit S6 kinase 1 activity over S6K2 activity (not, for example, an inhibitor of mTOR, which would result in the inhibition of S6K2, which is preferably avoided) for use in treating individuals suffering from or at risk of developing insulin resistance or diabetes are provided.

Diabetes as used herein refers to Type II diabetes resulting, for example, from obesity or overweight conditions resulting from fat accumulation, or from high circulating fatty acids. Therefore insulin resistance or diabetes resulting from high fat diets rather than conditions resulting in reduced pancreatic beta cells are intended.

S6K modulators (e.g., inhibitors) for use in treating insulin resistance or diabetes may be formulated as medicaments according to conventional methodology, depending on the exact nature of the modulator, and will typically comprise the modulator or a precursor thereof in association with a biologically acceptable carrier. In considering various therapies, it is understood that such therapies may be targeted to tissues demonstrated to express S6K1, in particular to adipose tissue, liver and muscle.

Compounds are administered at a dose that is therapeutically effective. The term "therapeutically effective amount" as used herein means that the amount of a compound (s) or pharmaceutical composition elicits a beneficial biological or medicinal response in a tissue, system, animal or human. For example, a therapeutically effective amount of an S6K1 inhibitory compound is a dose that leads to a clinically detectable improvement in insulin resistance or diabetes.

Treatment includes the management and care of an individual for the purpose of alleviating a symptom of insulin resistance. Treatment includes the administration of a compound to prevent the onset of symptoms or complications of the disorder, alleviating the symptoms or complications, or eliminating the condition or disorder.

Delivery of the modulator to the affected cells and tissues can be accomplished using appropriate packaging or administration systems. For example, the modulator may be formulated for therapeutic use with agents acceptable for pharmaceutical administration and delivered to the subject by acceptable routes to produce a desired physiological effect. An effective amount is that amount that produces the desired physiological effect, such as, reduced insulin resistance and type II diabetes.

In a further aspect of the invention, the invention also provides a modulator (e.g., selective inhibitor of S6 kinase 1) for the manufacture of a medicament for the treatment or prophylactic treatment of insulin resistance or diabetes according to claim 8.

The compositions may include, in addition to the above constituents, pharmaceutically-acceptable excipients, preserving agents, solubilizers, viscosity-increasing substances, stabilising agents, wetting agents, emulsifying agents, sweetening agents, colouring agents, flavouring agents, salts for varying the osmotic pressure, buffers, or coating agents. Such materials should be non-toxic and should not interfere with the efficacy of the active ingredient. The precise nature of the carrier or other material may depend on the route of administration. Examples of techniques and protocols can be found in "Remington's Pharmaceutical Sciences", 16th edition, Osol, A. (ed.), 1980.

Where the composition is formulated into a pharmaceutical composition, the administration thereof can be effected parentally such as orally, nasally (e.g. in the form of nasal sprays) or rectally (e.g. in the form of suppositories). However, the administration can also be effected parentally such as intramuscularly, intravenously, cutaneously, subcutaneously, or intraperitoneally (e.g. in the form of injection solutions).

Thus, for example, where the pharmaceutical composition is in the form of a tablet, it may include a solid carrier such as gelatine or an adjuvant. For the manufacture of tablets, coated tablets, dragees and hard gelatine capsules, the active compounds and their pharmaceutically-acceptable acid addition salts can be processed with pharmaceutically inert, inorganic or organic excipients. Lactose, maize, starch or derivatives thereof, talc, stearic acid or its salts etc. can be used, for example, as such excipients for tablets, dragees and hard gelatine capsules. Suitable excipients for soft gelatine capsules are, for example, vegetable oils, waxes, fats, semi-solid and liquid polyols etc. Where the composition is in the form of a liquid pharmaceutical formulation, it will generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may also be included. Other suitable excipients for the manufacture of solutions and syrups are, for example, water, polyols, saccharose, invert sugar, glucose, trihalose, etc. Suitable excipients for injection solutions are, for example, water, alcohols, polyols, glycerol, vegetable oils, etc. For intravenous, cutaneous or subcutaneous injection, or intracatheter infusion into the brain, the active ingredient will be in the form of a parenterally-acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers and/or other additives may be included, as required.

The present inventors have shown that wild type mice fed a high fat diet have strikingly elevated S6K1 activity (see Example 9). The invention therefore also provides a method of diagnosing a predisposition to insulin resistance or diabetes, comprising: in a sample from mammal detecting the level of S6 kinase, preferably S6 kinase 1, in the sample and correlating a change in the amount of S6 kinase in the sample when compared to a normal control value or range of values with a predisposition to insulin resistance or diabetes according to claim 9. The presence of S6 kinase can be easily determined using antibodies or using activity assays as described above. S6K expression can also be detected at the transcript level, e.g., using PCR techniques. When protein levels are detected, antibodies specific for S6K2 can be used as a control when S6K1 specific measurements are desired. In general, an increase in S6 kinase 1 activity of at least 10%, preferably at least 20%, 30%, 40% or 50% when compared to a normal control value or range of values is indicative of a predisposition to insulin resistance or diabetes. Most preferably, the increase in activity is at least 2- fold than that of a control value. The sample may be any tissue sample or body fluid, but is preferably adipose, muscle or liver tissue.

To determine the kinase activity of S6K 1 (independent of other S6K isoforms such as S6K2), a sample is obtained from a test subject. Cells present in the sample can be lysed and proteins extracted. Optionally, further purification steps can be carried out. The sample can then be subjected to immunoprecipitation using an S6K1 specific antibody, which are known in the art. Following immunoprecipitation of S6K1, a standard kinase assay is performed as described above.

The invention is further described, for the purposes of illustration only, in the following

### examples.

### EXAMPLES

Methods of molecular genetics, protein and peptide biochemistry and immunology referred to but not explicitly described in this disclosure and examples are reported in the scientific literature and are well known to those skilled in the art. For example, standard methods in genetic engineering are carried out essentially as described in Sambrook et al., Molecular Cloning: A laboratory manual, 2nd Ed., Cold Spring Harbor Laboratory Press, Cold Spring Harbor NY, 1989.

### Example 1:S6K1 deficient mice are smaller than wild type

S6K1 deficient mice were previously shown to have a reduction in body size during embryogenesis but the effect was thought to be mostly overcome by adulthood, diminishing from 20 to 15% by 11 weeks of age. This Example demonstrates that as they age, S6K1 deficient mice maintain a lower body weight relative to wild type mice. Male mice on a normal chow diet (NCD, 4% of total calories derived from fat, 3035 kcal kg-1, KLIBA-NAFAG, Switzerland) were followed over a period of seventeen weeks from ten weeks of age.

S6K1 deficient mice were generated as described by Shima et al. (1998, EMBO J., 17, 6649-6659). Mice were kept on a hybrid background derived from the C57BI/6 and 129Oia mouse strains, housed in groups of 12 (in cages of 3) and maintained on a 12 hour light/12 hour dark cycle (lights on at 06:00 GMT). Body weight was recorded weekly in wild type (wt) and S6K1 deficient mice fed regular chow diet. Unexpectedly, the results showed that the rate at which S6K1-/- mice gained weight was much slower than that of wild mice, such that the difference in weight at twenty-seven weeks, as compared to ten weeks, had increased to 25%. It should also be noted S6K1-/- mice displayed little variation in weight as compared to wild type (wt) mice.

### Example 2:S6K1 deficient mice have reduced body fat

Mice were dissected to determine the cause of the lower body weight exhibited by S6K1 deficient mice described in Example 1. S6K1 deficient mice were shown to have less intra-abdominal fat pads. Each fat mass and organ mass was weighed from tissue removed from 6 -month old male mice. Dissection of S6K1 mice revealed a severe reduction of epididymal white adipose tissue fat relative to wild type mice (0.8% +/- 0.1 % compared with 3.4% +/- 0.1 %; the values are averaged values +/- standard error mean; S.E.M). Percentage brown adipose tissue/body weight was also reduced in S6K1 deficient mice (0.5% +/- 0.05 % compared with 1.0 +/- 0.1 %), whereas organ size was essentially unaffected. Similar results were found also in female mice. The decrease in fat was not associated with a selective decrease in the deposition of fat in liver or muscles.

The results establish that S6K1 -/- mice have reduced body white fat and brown fat relative to wild type mice.

### Example 3: S6K1 deficient adipocytes are smaller

To establish why S6K1 deficient mice exhibit less fat, adipose tissue sections were stained with hematoxylin and eosin and visualized by 20-fold magnification using a histology microscope. Both epididymal white adipose tissue (WAT) and brown adipose tissue from S6K1 deficient mice exhibited smaller cell size when compared to wild type.

Analysis of the size of fat cells in epididymal fat pads by either scanning electron microscopy or by hematoxyline-eosine staining showed a striking reduction in cell size, with quantitation revealing that adipocytes from S6K1-/- mice were 71% smaller than those of wild type mice (wt: 3129 ± 904 , n=3; S6K1-/-: 918 ± 189 mm2 , n=3, P< 0.05), with many adipocytes exhibiting a multilocular phenotype. These studies also revealed that the distribution of cell size, like body weight, was much more homogeneous in S6K1-/- mice as compared to wild type mice and a rough calculation of cell surface areas versus total mass suggested that there was little effect on cell number. In summary, results from electron microscopy, histology and cell density/size analysis establishes that the reduction in fat in S6K1 deficient animals is due to a reduction in fat cell size.

### Example 4: Diet is not the cause of less fat in S6K1 deficient mice

To establish whether S6K1 mice exhibited dietary differences over wild type mice, which would explain the reduction in fat in S6K1 mice, food intake per mouse was measured every other day for 15 days using normal or high fat chow. Irrespective of whether placed on normal or high fat diet, S6K1 deficient mice eat the same total amount of food as wild type (about 4.6 +/- 0.1 g food/mouse/day), but compared to body weight, they eat much more (about 17% or more, or 0.18 compared to 0.15 g food/g body weight/day).

Furthermore, despite their apparent leanness, the mice did not appear to be starving as glucose homeostasis appeared normal (Table I below), consistent with the fact that there was no increase in ketone body formation (D-β-hydroxybutyrate values 1.3 mg/dl ± 0.05 for wild type mice, n=8; 1.4 mg/dl ± 0.1 for *S6K1^{-l-}* mice, n=7; P= 0.8).

### Example 5: S6K1 deficient mice exhibit an enhanced metabolic rate

The increased food uptake, combined with reduced WAT, raised the possibility of enhanced metabolic activity. The metabolic rate of S6K1 deficient and wild type mice were examined by indirect calorimetry to monitor oxygen consumption and carbon dioxide production every 15 min over an eight-hour fasting period employing an Oxymax (Columbus Instruments, Columbus, OH).

The results show a striking 27% increase in the rate of oxygen consumption in S6K1-/- mice versus wild type mice through out the experiment. Calculation of the respiratory exchange ratio (RER = ratio of CO2 produced to 02 consumed) gave values of 0.713 ± 0.004 for wild type mice and 0.709 ± 0.003 for S6K1-/- mice; P< 0.01), arguing that both animals were largely utilizing fatty acids as an energy source.

Metabolite assays were carried out as follows. Blood was collected from retroorbital sinus after overnight fast or 1 hr after beginning of the meal followed by overnight fast. Nonesterified fatty acids, and triglycerides were determined by enzymatic assays (Boehringer-Mannheim, Germany). Plasma leptin was measured using Rat leptin RIA kit (Linco Research, St Louis, MO.). The results are shown in Table I. Plasma leptin levels were significantly reduced in S6K1-/- mice (Table I) in keeping with the mice's increased consumption of food relative to body weight.

**Table I one-hour postprandial insulin, triglycerides, free fatty acids and leptin levels after overnight fasting of 6 month old male wild type, and S6K1 deficient mice fed a normal, or high fat diet.**

| | WT | | S6K1 -/- | |
|---|---|---|---|---|
| Diet | Normal | High Fat | Normal | High Fat |
| Insulin (µg/l) | 0.57 ± 0.09 | 1.91 ± 0.78 | 0.38 ± 0.08* | 0.22 ± 0.06* |
| Triglycerides(mmol/l) | 0.61 ± 0.09 | 1.08 ± 0.15 | 0.55 ± 0.06 | 0.95 ± 0.14 |
| Free fatty acids (mmol/l) | 0.26 ± 0.05 | 0.27 ± 0.02 | 0.19 ±0.04 | 0.56 ± 0.10* |
| leptin (ng/ml) | 5.50 ± 0.82 | 12.6 ± 0.00 | 3.42 ± 0.75 | 6.34 ± 2.35* |

| | | | | |
|---|---|---|---|---|
| Data represent means ± s.e.m. *, P< 0.05 compared with wild type (n=6 to 18). P values were calculated by two-tailed, unpaired Student's *t*-test. | | | | |

Although not wishing to be bound by theory, given the reduced adipose tissue mass, increased metabolic rate, and the fact that when corrected for body weight, plasma triglycerides and free fatty acids were similar between genotypes (Table I), it was reasoned that in the absence of S6K1, either triglycerides in adipose tissue were being rapidly utilized or that the free fatty acids never reached adipose tissue for storage, but were immediately taken up by muscle and oxidized.

Although WAT is not normally an energy consuming tissue, that there was no difference in the basal levels of circulating fatty acids in S6K1-/- mice, as compared to wild type mice (Table I), suggested that free fatty acids may be directly oxidized in WAT. Consistent with this hypothesis, electron micrographs revealed the presence of many multilocular adipocytes, which displayed a dramatic increase in the size and number of mitochondria, phenotypes completely absent in adipocytes from wild type mice. Others have shown that overexpression of UCP1 in WAT induces a similar phenotype, and UCP1 levels, measured by quantitative real time-PCR, were dramatically upregulated in WAT from S6K1-/- mice as compared to WAT from wild type mice.

### Example 6: S6K1 deficient mice exhibit increased basal lipolysis

The breakdown of triglycerides in adipose tissue (lipolysis) was measured by monitoring the release of either fatty acids or glycerol from mature adipocytes. Primary adipocytes were prepared from epididymal fat pads by collagenase digestion as described previously (Marette et al., 1991). Cells were incubated for 30 min at 37°C with or without norepinephrine (Sigma-Aldrich SARL, St-Quentin Fallavier, France) at different concentrations (10⁻⁸ to 10⁻⁵M). Norepinepherine is a beta-adrenergic agonist and stimulates the breakdown of adipocyte triglyceride to glycerol and free fatty acids (lipolysis) and increases the basal metabolic rate (thermogenesis). Despite the reduction in cell size of epididymal adipocytes (see Example 3), the results showed that the basal rate of fatty acid release was approximately 5-fold higher in adipocytes from S6K1-/- mice as compared to wild type mice. The rate of fatty acid release increased in both genotypes in a dose dependent manner upon the addition of norepinephrine, reaching similar maximum values, with the increase much steeper in wild type mice. Similar results were obtained for the release of glycerol. Therefore, S6K1-/- mice are protected against fat accumulation partly due to a sharp increase in basal lipolysis.

### Example 7: S6K1 deficient mice exhibit impaired adipogenesis

During the isolation of mature adipocytes for the lipolysis studies, it became evident that there were few preadipocytes present in epididymal WAT of S6K1-/- mice. This, along with the inability of adipocytes to store triglycerides, prompted experiments to compare the ability of mouse embryonic fibroblasts (MEFs) from S6K1-/- and wild type embryos to differentiate into adipocytes using an adipocyte differentiation mix.

Briefly, adipocyte differentiation was induced essentially as previously described (Hansen et al., 1999, J. Biol. Chem., 274, 2386-2393) using wild type or S6K1 deficient mouse embryonic fibroblasts (MEFs). The passage number of MEFs was within one passage. For differentiation, 2-day post confluent cells (day 0) were treated with growth medium containing 1 µM dexamethasone (Sigma), 0.5 mM methylisobutylxanthine (Aldrich), 5 µg/ml insulin (Boehringer Mannheim), and Ciglitazone (thiazolinedione, PPAR agonist: BIOMOL, GR-205, 0.5 µM) for 2 days. From day 2, the medium contained 5 µg/ml insulin and Ciglitazone and renewed every other day. Oil red O staining: Oil red O staining solution (0.5% Oil red O in isopropyl alcohol solution-distilled water (60:40) was filtered. Cells were washed with PBS and stained for 30 min and then washed with distilled water two times. Cells deficient in S6K1 showed much less staining. Therefore, MEFs lacking S6K1 had a reduced adipogenic potential, as evaluated by the reduced Oil Red O lipid staining, consistent with lower levels of aP2 mRNA. Taken together the results suggest that S6K1-/- mice have reduced WAT because of impaired adipogenesis and an inability to store fat.

### Example 8: S6K1 deficient mice are protected against diet-induced obesity

The failure of S6K1-/- mice to accumulate fat with age combined with their overall increase in metabolic rate suggested that they may be protected against diet-induced obesity. In this example, body weight was recorded weekly in wild type and S6K1 deficient mice fed high fat diets (HFD, 60% of total calories derived from fat, 4057 kcal kg-1, Research diets, USA). When S6K1 mice are placed on a high fat diet, absolute body weight gain of S6K1 deficient mice was about 10.5 g over the period of high fat diet feeding (from week 7 to 27 of age) compared to about 14.4 g in wild type mice. Similar to the situation on the NCD (Example 1), S6K1-/- mice displayed little variation in weight on a HFD as compared to wild type mice. Given the smaller body size of the knockout, relative weight gain was similar between genotypes (58.9% increase of body weight in S6K1 deficient mice compared to 58.0% increase of body weight in wild type after high fat diet feeding for 5 months. Even though the relative percentage of body weight gain in S6K1 deficient mice was similar to wild type, they fail to put on fat to the same extent as wild type mice. Over a three-month period, wild type mice gained 0.1 g/g compared with 0.02 g/g fat/body weight by S6K1 deficient mice.

Mice of both genotypes consumed less food on a HFD than on a NCD, probably due to the higher caloric density of the HFD as compared to the NCD. Although in absolute terms, S6K1-/- mice consume the same amount of food as wild type mice, when normalized to body weight they consume 44% more food. Thus, even though S6K1 mice eat more, they do not put on fat to the same degree as wild type mice.

Indirect calorimetry measurements were conducted over an eight-hour fasting period on HFD and NCD mice. In both genotypes oxygen consumption increased on the HFD, as compared to the NCD, but the effect was more pronounced for S6K1-/- mice, such that the difference between S6K1-/- mice and wild type mice increased from 25% to 30%. The data further showed that the RER remained unchanged in S6K1-/- mice on a HFD versus a NCD, 0.708 ± 0.002 vs 0.709 ± 0.004, respectively, whereas in wild type mice the RER increased from 0.713 ± 0.004 on NCD to 0.729 ± 0.002 (n=6, P< 0.01) on a HFD diet, indicative of an increase in carbohydrate relative to fatty acid oxidation. Despite the fact that S6K1-/- mice on either diet displayed a high metabolic rate, on a HFD they exhibited a threefold increase in circulating free fatty levels, whereas in wild type mice there was no significant change in free fatty acids levels (Table I). Hence, S6K1-/- mice fail to accumulate fat at an appreciable rate when challenged with a HFD.

### Example 9: Obese animals and wild type animals on high fat diet exhibit elevated S6K1 phosphorylation in adipose tissue

To examine whether S6K1 is affected in adipose tissue from normal and obese genetic models, the phosphorylation of S6K1 was detected. This can easily be carried out using phospho-specific antibodies. The level of S6K1 T389 and S6 S240/S244 phosphorylation in adipose tissue of wild type mice fasted for a short period was determined. Upon growth factor stimulation, S6 is multiply phosphorylated at the carboxy terminus on five serine residues in an ordered fashion beginning with Ser236, followed sequentially by > Ser235 > Ser240 > Ser244 and Ser247. Basal values of S6K1 T389 and S6 S240/S244 phosphorylation are low in mice fasted for a short period after being maintained on a NCD. In sharp contrast, the same mice maintained on a HFD and treated under the identical conditions, maintained high-elevated levels of S6K1 T389 and S6 S240/S244 phosphorylation.

The present inventors also examined S6K1 activity in ob/ob mice, a genetic model for obesity. The results show that the ob/ob mice maintained on NCD have elevated S6K1 T389 and S6 S240/S244 phosphorylation as compared to wild type mice on a NCD. Preliminary human data are consistent with these data and provide further support for S6K1 as a promising drug target in the treatment of patients suffering from obesity and as a potential diagnostic marker.

### Example 10: Mature S6K1 deficient mice do not exhibit insulin resistance

S6K1 deficient mice have previously been suggested to be hypersensitive to insulin in their peripheral tissues, because they maintain normal fasting glucose levels, despite their innate hypoinsulinemia and mild glucose intolerance (Pende et al., 2000).

Glucose tolerance tests and in vivo insulin secretion were performed as previously described (Pende et al., 2000). Insulin tolerance tests were performed by intraperitoneal injection of 0.75 U/kg body weight insulin after 3-h fast. Blood was collected before injection and 15, 30, 60 and 90 min after injection.

On a Normal Chow Diet, more mature S6K1 deficient mice exhibit a slight tendency towards increased insulin sensitivity versus wild type mice, as indicated by the moderately faster rate of glucose clearance upon insulin tolerance testing. However, the effect is small. This raised the likelihood that S6K1 deficient mice fed on a High Fat Diet would like wild type mice become insulin resistant if fed on a High Fat Diet.

Unexpectedly, the results of such an analysis show, that despite a sharp increase in free fatty acids (Table 1), which is implicated in the etiology of insulin resistance, S6K1 deficient mice remain insulin sensitive, whereas wild type mice display strong insulin resistance as expected on a high fat diet. Consistent with this, wild type mice become glucose intolerant on a high fat diet as compared to a match set of mice on a normal chow diet. However, S6K1 deficient mice on high fat diet remain glucose tolerant, despite a further significant decrease in postprandial (1 h) circulating insulin levels (Table1).

### Example 11: Mechanism of insulin sensitivity in S6K1 deficient mice on high fat diet

This Example addresses how S6K1 deficient mice remain hypersensitive to insulin in their peripheral tissues on a high fat diet.

After a 6 hour fast, mice were anesthetized and 0.75Ukg-1 insulin (Eli Lilly) or an equal volume of vehicle was administered by i.v. injection. Liver, adipose (Epididymal fat pads) and muscle (Gastrocnemius) were collected in liquid nitrogen 5 m inutes after injection. Tyrosine phosphorylation of insulin receptor was measured in liver. Protein extracts (1 mg) from tissue samples were prepared for immunoprecipitation and analyzed as described (Hirosumi, 2002). Antibodies were purchased from Santa Cruz (anti-insulin receptor β) , Upstate Biotechnology (anti-phosphotyrosine) and Cell Signaling (anti-PKB, anti-phospho PKB-Ser473, anti-phosphoS6K-Thr 389, anti-phospho S6 240/244, and Upstate Biotechnology (anti-phosphotyrosine).

Examination of PKB activity in adipose tissue following insulin injection, as a reporter for the insulin signaling pathway, revealed that the activation of the kinase was suppressed in wild type mice maintained on a high fat diet versus mice raised on a normal chow diet. However, in contrast to wild type mice, there was no significant difference in PKB activation in S6K1 deficient mice, regardless of the diet. The absence of an effect of high fat diet on PKB activation in S6K1 ko mice was also true for liver and muscle.

Examination of insulin receptor autophosphorylation shows that it is also strongly suppressed by high fat diet in the liver of wild type mice, but not in S6K1-/- mice, raising the possibility that the insulin receptor may be the target of the negative feedback loop.

Given these findings, it raised the possibility that on a high fat diet, S6K1 activity is elevated and that this enhanced activity is responsible for inducing insulin resistance. To test this possibility the present inventors examined the level of S6K1 T389 phosphorylation and S6 phosphorylation in fat and muscle, five minutes following an intravenous administration of insulin. The results show that basal values of S6K1 T389 phosphorylation are low in both high fat diet and normal chow diet animals, but in contrast to PKB Ser 473 phosphorylation, insulin stimulates these levels even higher, potentially suppressing insulin signalling further.

These findings raised the possibility that a potential mechanism by which obese humans become insulin resistant is through nutrient induced S6K1 activation, suppressing insulin signaling through a negative feedback loop. To test this possibility the present inventors examined S6K1 activity in patients, which were clinically lean, obese or obese and diabetic following a six-hour fasting period. The results show that the obese and the obese/diabetic patients have elevated S6K1 levels as compared to lean patients, consistent with the feedback loop in which S6K1 is a major negative effector in insulin signaling.

The results taken together strongly suggest that S6K1 may be a potential target for drug intervention in the treatment of patients suffering from obesity-induced diabetes or patients suffering from insulin resistance in their peripheral tissues.

## Claims

1. A method of identifying an agent effective in treating insulin resistance, said method comprising the steps of:
i) incubating S6 kinase with a compound;
ii) detecting S6 kinase activity; and
iii) determining a compound-induced modulation in the S6 kinase activity relative to when said compound is absent, wherein an inhibition of the S6 kinase 1 activity in the presence of the compound is indicative of an agent effective in treating insulin resistance.

2. The method of of claim 1, comprising determining S6 kinase activity using S6 as a substrate,

3. The method of of claim 1, comprising determining S6 kinase activity using a peptide as a substrate.

4. A method of screening for an agent effective in treating insulin resistance, the method comprising
(a) contacting transcriptionally active cellular components in vitro with a nucleic acid encoding an S6 kinase 1 gene operably linked to a promoter sequence or an S6 kinase 1 promoter sequence operably linked to a reporter gene in the presence of at least one compourd; and
(b) detecting an effect of said compound on S6 kinase 1 expression or S6 kinase 1 promoter activity, wherein detection of a decrease in S6 kinase 1 expression or promoter activity is indicative of an agent effective in treating insulin resistance.

5. The method of claim 4, wherein said transcriptionally active cellular components and said nucleic acid is present in a cell.

6. The method of claim 4 or 5, further comprising detecting an effect of said agent on insulin resistance.

7. A method for reducing insulin resistance, said method comprising contacting in vitro an adipocyte, myocyte or hepatocyte with an effective amount of an S6 kinase 1 inhibitor, selected from the group consisting of antibody or antibody fragment specific for S6 kinase 1, and antisense, ribozyme or siRNA that preferentially reduces expression of S6 kinase 1 compared to S6 kinase 2

8. Use of a substance for the manufacture of a medicament for treating or preventing the development of insulin resistance or diabetes, wherein said substance is an S6 kinase inhibitor selected from the group consisting of antibody or antibody fragment specific for S6 kinase 1, and antisense, ribozyme or siRNA that preferentially reduces expression of S6 Kinase 1 compared to S6 kinase 2.

9. A method of diagnosing insulin resistance or a predisposition to insulin resistance, comprising:
(a) detecting the level of S6 kinase activity in a sample from a mammal; and
(b) correlating a change in S6 kinase activity when compared to a normal control value or range of values with insulin resistance or a predisposition to insulin resistance, wherein an increase in the level of S6K1 activity compared to a normal control indicates that said mammal is suffering from or has a predisposition to developing insulin resistance.

## Patentansprüche

1. Verfahren zur Identifikation eines Mittels, das zur Behandlung von Insulinresistenz wirksam ist, wobei das Verfahren die folgenden Schritte umfasst:
i) das Inkubieren von S6-Kinase mit einer Verbindung;
ii) das Nachweisen der S6-Kinase-Aktivität; und
iii) das Bestimmen einer durch die Verbindung induzierten Hemmung der S6-Kinase-Aktivität verglichen mit der Abwesenheit der Verbindung, wobei eine Hemmung der S6-Kinase 1-Aktivität in Anwesenheit der Verbindung auf ein Mittel hindeutet, das zur Behandlung von Insulinresistenz wirksam ist.

2. Verfahren nach Anspruch 1, umfassend die Bestimmung der S6-Kinase-Aktivität unter Verwendung von S6 als Substrat.

3. Verfahren nach Anspruch 1, umfassend die Bestimmung der S6-Kinase-Aktivität unter Verwendung eines Peptids als Substrat.

4. Verfahren für das Screening nach einem Mittel, das zur Behandlung von Insulinresistenz wirksam ist, wobei das Verfahren umfasst:
(a) das Zusammenbringen transkriptionell aktiver zellulärer Bestandteile in vitro mit einer Nukleinsäure, die für ein S6-Kinase 1-Gen, das betriebsfähig mit einer Promotorsequenz verbunden ist, oder für eine S6-Kinase 1-Promotorsequenz codiert, die betriebsfähig mit einem Reportergen verbunden ist, in Anwesenheit mindestens einer Verbindung; und
(b) das Nachweisen der Wirkung der Verbindung auf die S6-Kinase 1-Expression oder die S6-Kinase 1-Promotoraktivität, wobei der Nachweis einer Abnahme der S6-Kinase 1-Expression oder -Promotoraktivität auf ein Mittel hindeutet, das zur Behandlung von Insulinresistenz wirksam ist.

5. Verfahren nach Anspruch 4, wobei die transkriptionell aktiven zellulären Bestandteile und die Nukleinsäure in einer Zelle vorliegen.

6. Verfahren nach Anspruch 4 oder 5, das außerdem das Nachweisen einer Wirkung des Mittels auf die Insulinresistenz umfasst.

7. Verfahren zur Verringerung von Insulinresistenz, umfassend das Zusammenbringen einer Adipozyte, Myozyte oder Hepatozyte in vitro mit einer wirksamen Menge eines S6-Kinase 1-Inhibitors, ausgewählt aus der Gruppe, bestehend aus für S6-kinase 1 spezifischem Antikörper oder Antikörperfragment und Antisense-, Ribozym- oder siRNA, der/das/die bevorzugt die Expression von S6-Kinase 1 verglichen mit S6-Kinase 2 verringert.

8. Verwendung einer Substanz zur Herstellung eines Arzneimittels zur Behandlung oder Vorbeugung der Entwicklung von Insulinresistenz oder Diabetes, wobei die Substanz ein S6-Kinase-Inhibitor ist, ausgewählt aus der Gruppe, bestehend aus für S6-Kinase 1 spezifischem Antikörper oder Antikörperfragment und Antisense-, Ribozym- oder siRNA, der/das/die bevorzugt die Expression von S6-Kinase 1 verglichen mit S6-Kinase 2 verringert.

9. Verfahren zur Diagnose von Insulinresistenz oder einer Prädisposition für Insulinresistenz, umfassend:
(a) das Nachweisen des Spiegels an S6-Kinase-Aktivität in einer Probe von einem Säuger, und
(b) Korrelieren einer Veränderung in der S6-Kinase-Aktivität verglichen mit einem normalen Kontrollwert oder Bereich von Werten mit Insulinresistenz oder einer Prädisposition für Insulinresistenz, wobei ein Anstieg im Spiegel der S6K1-Aktivität verglichen mit einer normalen Kontrolle anzeigt, dass der Säuger an Insulinresistenz leidet oder eine Prädisposition für die Entwicklung von Insulinresistenz hat.

## Revendications

1. Procédé d'identification d'un agent efficace dans le traitement de l'insulinorésistance, ledit procédé comprenant les étapes consistant à :
i) incuber la kinase S6 avec un composé ;
ii) détecter l'activité kinase S6 ; et
iii) déterminer une modulation induite par le composé de l'activité kinase S6 par rapport à lorsque le composé est absent, **caractérisé en ce qu'**une inhibition de l'activité kinase S6 1 en présence du composé est indicatrice d'un agent efficace dans le traitement de l'insulinorésistance.

2. Procédé de la revendication 1, comprenant la détermination de l'activité kinase S6 en utilisant S6 en tant que substrat.

3. Procédé de la revendication 1, comprenant la détermination de l'activité kinase S6 en utilisant un peptide en tant que substrat.

4. Procédé de criblage pour rechercher un agent efficace dans le traitement de l'insulinorésistance comprenant les étapes consistant à
(a) mettre en contact des composants cellulaires transcriptionnellement actifs in vitro avec un acide nucléique codant pour un gène de kinase S6 1 fonctionnellement lié à une séquence de promoteur ou une séquence de promoteur de kinase S6 1 fonctionnellement liée à un gène rapporteur en présence d'au moins un composé ; et
(b) détecter un effet dudit composé sur l'expression de kinase S6 1 ou l'activité du promoteur de kinase S6 1, **caractérisé en ce que** la détection d'une diminution de l'expression ou de l'activité du promoteur de kinase S6 1 est indicatrice d'un agent efficace dans le traitement de l'insulinorésistance.

5. Procédé de la revendication 4, **caractérisé en ce que** lesdits composants cellulaires transcriptionnellement actifs et ledit acide nucléique sont présents dans une cellule.

6. Procédé de la revendication 4 ou 5, comprenant en outre la détection d'un effet dudit agent sur l'insulinorésistance.

7. Procédé pour réduire l'insulinorésistance, ledit procédé comprenant la mise en contact in vitro d'un adipocyte, un myocyte ou un hépatocyte avec une quantité efficace d'un inhibiteur de kinase S6 1, choisi dans le groupe constitué d'un anticorps ou d'un fragment d'anticorps spécifique de la kinase S6 1, et un antisens, ribozyme ou ARNsi qui réduit préférentiellement l'expression de la kinase S6 1 par rapport à la kinase S6 2.

8. Utilisation d'une substance pour la fabrication d'un médicament pour traiter ou prévenir le développement de l'insulinorésistance ou du diabète, **caractérisée en ce que** ladite substance est un inhibiteur de kinase S6 choisi dans le groupe constitué d'un anticorps ou d'un fragment d'anticorps spécifique de la kinase S6 1, et un antisens, ribozyme ou ARNsi qui réduit préférentiellement l'expression de la kinase S6 1 par rapport à la kinase S6 2.

9. Procédé de diagnostic de l'insulinorésistance ou d'une prédisposition à l'insulinorésistance, comprenant les étapes consistant à :
(a) détecter le taux d'activité kinase S6 dans un échantillon provenant d'un mammifère ; et
(b) corréler un changement de l'activité kinase S6 par rapport à une valeur ou plage de valeurs témoin normale à l'insulinorésistance ou une prédisposition à l'insulinorésistance, **caractérisé en ce qu'**une augmentation du taux d'activité S6K1 par rapport à un témoin normal indique que ledit mammifère souffre de ou a une prédisposition au développement d'une insulinorésistance.
